# EUROPEAN PATENT SPECIFICATION

(11) **EP 1 372 702 B1**
(45) Date of publication and mention of the grant of the patent: **18.07.2007**
(21) Application number: 01920006.2
(22) Date of filing: 03.04.2001
(51) Int. Cl.: A61K 38/21, A61P 9/10

(54) **TREATMENT OF HYPOXIA/ISCHAEMIA RELATED BLOOD FLOW RESISTANCE USING BETA-INTERFERON**
BEHANDLUNG VON HYPOXYA/ISCHAEMIA BLUTFLUSSWIDERSTAND MIT BETA-INTERFERON
TRAITEMENT DE L'INSUFFISANCE CIRCULATOIRE LIEE A L'HYPOXIE/ISCHEMIE AVEC DE L'INTERFERON-BETA

(43) Date of publication of application: 02.01.2004
(73) Proprietor: U-Cytech B.V., 3584 CM Utrecht (NL)
(72) Inventor: VELDHUIS, Wouter, Bernard, 3512 JP Utrecht (NL); VAN DER MEIDE, Petrus, Hendrikus, NL-2631 HT Nootdorp (NL); NICOLAY, Klaas, NL-3992 CM Houten (NL)
(74) Representative: Prins, Adrianus Willem
(86) International application number: PCT/NL2001/000276
(87) International publication number: WO 2002/080959

(56) References cited:
- EP-A- 0 797 998
- PATENT ABSTRACTS OF JAPAN vol. 1997, no. 10, 31 October 1997 (1997-10-31) & JP 09 151137 A (TORAY IND INC), 10 June 1997 (1997-06-10)

## Description

The invention relates to the field of medicine. The invention more in particular relates to the treatment of diseases, more specifically diseases in which interruption of blood flow and/or activation/infiltration/proliferation of immune cells is detrimental.

Interruption of blood flow is detrimental to tissue and in fact is the cause of a variety of human disease. Restoration of blood flow, spontaneous and/or treatment-induced can theoretically salvage all or part of the affected tissue. The success of this reperfusion in preventing further tissue damage is amongst others dependent on the severity of blood flow decrease and the time to restoration of blood flow to levels compatible with cell survival.

Treatment directed to increase blood flow - and/or spontaneous restoration of blood flow - to therapeutically relevant levels is often counteracted by a phenomenon that we refer to as hypoxia/ischaemia (H/I) related flow resistance. It is very often observed that the microvasculature downstream of the site of obstruction responds by resisting the increased blood flow following removal of the obstruction. The H/I related flow resistance counteracts the quality of tissue perfusion after removal of the primary obstruction, or, when the obstruction has not (yet) been removed: the quality of the remaining perfusion. This phenomenon is not only relevant in situations where blood supply is temporarily interfered with. A similar phenomenon is observed in situations where the demand for blood flow is chronically lower than the supply. Although the term hypoxia refers to situations of oxygen shortage, the phenomenon does not have to be related to the oxygen shortage. The term is here only used to indicate a situation in or after which the phenomenon is observed. H/I related flow resistance is a problem in a variety of human disease. Various associations and explanations for the resistance have been proposed, however as of yet this has not led to the development of improved therapies.

The present invention now provides the use of interferon-β or a functional part, derivative and/or analogue thereof, for the preparation of a medicament for the treatment of H/I related blood flow resistance in an individual. Said interferon-β or a functional part, derivative and/or analogue thereof, leads to reduced H/I related flow resistance in said individual. Perfusion of tissue displaying H/I related blood flow resistance in the untreated individual, is improved in the comparable tissue in the treated individual. Improved perfusion results in less damage, better recovery or improved repair of the affected tissue. H/I related blood flow resistance does not have to be displayed by the individual. Prophylactic application is also beneficial. I.e. when it is expected that H/I related blood flow resistance can occur in an individual said individual can be treated with a method of the invention and profit from the resulting improved perfusion as compared to the situation where no interferon-β or functional part, derivative and/or analogue thereof, is given to said individual. As a non-limiting example this situation is illustrated for instance in surgery where one or more parts of the body may suffer from reduced blood flow due to isolation from the circulation. With the phrase "treatment of H/I related blood flow resistance" is meant that said blood flow resistance is at least in part reduced as a result of said treatment. Complete disappearance of the blood flow resistance is possible but not required in the present invention.

H/I related blood flow resistance can occur in many situations. A person that has not been able to breath oxygen for a limited amount of time, for instance, because the individual is in a situation where he/she has to be resuscitated, can be treated with a method of the invention and expect a beneficial effect on the overall perfusion. In a preferred embodiment of the invention said H/I related blood flow resistance is the result of a shortage in blood supply. A situation where H/I related blood flow resistance can occur is very frequently a local problem. Therefore typically, said H/I related blood flow resistance is restricted to a part of the body of said individual, preferably due to an obstruction of a blood vessel. An obstruction can be a complete obstruction of a vessel allowing no passage of blood. An obstruction can also be an incomplete obstruction of a vessel that disables the flow of blood to meet the demand of blood flow. An obstruction can be in the form of a clot that clogs the vessel. However, an obstruction can also be the closing of a vessel by force exerted from the outside for instance due to clasping. An obstruction can also be caused by a cut effectively interfering with proper blood flow.

In one aspect the invention therefore provides the use of interferon-β or a functional part, derivative and/or analogue thereof, for the preparation of a medicament for the treatment of a H/I related blood flow resistance in an individual, said individual comprising at least one blood vessel obstruction causing ischaemia (the supply of blood lags behind the demand of blood, whether due to obstructions, increased demand, lowered level of oxygen and/or nutrients in the blood or any other way) in tissue that is situated downstream from said obstruction. A treatment with said medicament preferably results in improved survival of cells in said individual, preferably, said cells comprises neuronal cells. Also described herein is a method for at least in part improving blood flow in post-ischemic tissue comprising administering to an individual, having said tissue, β-interferon or a functional part, derivative and/or analogue thereof. Further described is a method for at least in part preventing cell death in post-ischemic tissue comprising administering to an individual, having said tissue, β-interferon or a functional part, derivative and/or analogue thereof. Preferably, said cell death is in part prevented in neuronal tissue.

In a preferred aspect of the invention said H/I related blood flow resistance is restricted to the brain, the heart, a transplanted organ and/or a limb. The invention can be used to improve survival of transplanted organs or parts thereof. In organ transplantation, reperfusion injury is an inflammatory cell-mediated response that causes tissue damage immediately following transplantation. This process has been implicated in the development of acute and chronic rejection of transplants. NF-kB is a transcription factor that upregulates adhesion molecules ICAM-1, VCAM-1, and E-selectin following reperfusion. Systemic treatment with sulfasalazine, a potent inhibitor of NF-kB, was shown to decrease adhesion molecule expression, decrease reperfusion injury, and prolong allograft survival in rat cardiac transplants. β-interferon reduces the expression of adhesion molecules, eg VCAM-1, and can be administered to an individual according to a method of the invention to prolong allograft survival.

In a preferred embodiment, said H/I related blood flow resistance is restricted to a part of the brain and/or the heart. Particularly the brain is preferred because the administration of β-interferon or a functional part, derivative and/or analogue thereof, has a dramatic effect on H/I related flow resistance in this part of the body. Administration results in significantly less neuronal damage, because blood flow in the affected area is increased significantly compared to untreated individuals. Due to this effect the volume of the damaged area in the brain as a result of the administration is significantly reduced compared to untreated. In a preferred embodiment β-interferon or a functional part, derivative and/or analogue thereof, is administered to patients in which the blood flow is insufficient in an area of the brain, preferably as the result of a stroke or trauma. In cases where an obstruction of a vessel is the reason for the insufficient blood flow it is preferred that the compound is administered as soon as possible, and preferably even before occurrence of said obstruction. Administration can even prolong the time window in for the (partial) removal of the obstruction. In this case administration is continued until a more or less stable situation has been arrived at. In cases where hypoxic/ischaemic tissue is reperfused, or perfused to a larger extend, a stable situation typically is arrived at within one week after the increased supply of blood flow was initiated.

It is thought that in the brain, ischaemia and trauma elicit an inflammatory response in which mediators (cytokines, chemokines and adhesion molecules) are released and immune cells infiltrate the injured brain. Upregulation of pro-inflammatory cytokines, chemokines and endothelial-leukocyte adhesion molecules starts soon after focal ischemia and trauma and continues during the evolution of tissue injury. For leukocytes to gain access to their target tissues, a variety of signaling proteins and adhesion molecules act in concert to permit chemotaxis, endothelial cell attachment and transmigration. For example a rapid overproduction of TNF-alpha leads to the stimulation of adhesion molecule expression with subsequent accumulation of leukocytes in the ischemic focus. Focal adhesion of leukocytes to the endothelium is a key step in inflammation and certain vascular disease processes. P- and E-selectin and ICAM-1, expressed by activated endothelium, and the leukointegrin CD11/CD18 expressed by activated leukocytes, have been shown to be crucial to this adhesion. E-selectin and ICAM-1 are synthesized by stimulation with cytokines such as IL-1 and TNF. Immunoreactivity to ICAM-1 and CD11/CD18 has been demonstrated after 60 min transient focal ischemia in the rat, in both core and penumbra, increasing from 3 to 24h after reperfusion. Leukocytic infiltration in this example was seen in the ischemic areas from 12 to 24h after reperfusion. The postulated effects of leukocytes in the pathogenenesis of cerebral ischemic damage are as follows: a reduction in cerebral blood flow by vessel plugging and/or release of vasoconstrictive mediators such as endothelin and exacerbation of BBB- or parenchymal injury via hydrolytic enzyme release, oxygen radical production and lipid peroxidation. IFN-B stimulates IL-10 prodcution which could in theory result in decreased TNF-alpha and IFN-gamma production.In traumatic brain injury (TBI), the dysfunction of the blood-brain barrier is mediated by intracerebral neutrophil accumulation, chemokine release (eg IL-8) and upregulation of adhesion molecules (eg ICAM-1, VCAM-1). In patients with severe TBI, it was found that elevated cerebrospinal fluid IL-8 and soluble (s)ICAM-1 correlated with BBB-dysfunction. IL-8 and sICAM-1 were upregulated for 19 days after severe TBI, whereas their stimulator TNF-alpha was upregulated for 9 days. The same holds true for critical limb ischemia. The present invention is also of use in individuals that are suffering from stroke or atherosclerosis in general. Adhesion of monocytes to the surface of intact endothelial cells appears to be an early event in the development of atherosclerotic lesions. The chemokines, monocyte chemoattractant protein-1 (MCP-1) and IL-8, are found in human atheroma, and mice lacking receptors for these chemokines are less susceptible to atherosclerosis and have fewer monocytes in vascular lesions. In vitro it was shown that MCP-1 and IL-8 cause monocytes to adhere to monolayers expressing E-selectin.

In the heart, coronary endothelial dysfunction after ischemia and reperfusion has been demonstrated. This endothelial dysfunction could be prevented by ischemic preconditioning. Experiments performed on cultured cells showed that the endothelial protection induced by preconditioning was due to a lesser expression of endothelial adhesion molecules such as ICAM-1, leading in vivo to a lesser adhesion of neutrophils to endothelial cells.

The reasonings given above are given only to provide the reader with a possible mechanism. The striking effects of β-interferon or a functional part, derivative and/or analogue thereof on blood flow as demonstrated in the present invention can also be caused through an entirely different mechanism.

In another aspect, the invention provides a use of β-interferon or a functional part, derivative and/or analogue thereof for the preparation of a medicament for the treatment of H/I related blood flow resistance. Preferably, said blood flow resistance is correlated with ischemia.

Also described herein is a use of β-interferon or a functional part, derivative and/or analogue thereof for the preparation of a medicament for the treatment of impairment of blood flow recovery. Preferably, said impairment is in a capillary vessel. Preferably, said impairment is in the brain. The invention further provides a use of β-interferon or a functional part, derivative and/or analogue thereof for the preparation of a medicament for at least in part preventing cell death in post-ischemic tissue.

The present invention also provides means and uses for at least in part preventing ischemia related damage to tissue. Reduction in tissue damage is, most likely, the result of reduced micro-vascular clogging, which occurs in situ in microvessels downstream from the site of occlusion. Reduced micro-vascular clogging prevents deterioration of blood flow in the microvasculature downstream of the ischemia upon the (partial) removal of the primary occlusion. Also described herein is a method for at least in part improving blood flow in tissue that has suffered or is suffering from ischemia, comprising administering to an individual, having said tissue, β-interferon or a functional part, derivative and/or analogue thereof. β-Interferon or a functional part, derivative and/or analogue thereof may be administered before the cause for the ischemia is removed. Preferably, said β-interferon or a functional part, derivative and/or analogue thereof is given as soon as possible, however, together or immediately after treatment given to (partially) remove the occlusion is also possible. In a preferred embodiment β-interferon or said functional part, derivative and/or analogue thereof is administered as soon as possible after the obstruction has occurred. In this way medication or other methods to remove the obstruction is given a larger time window for effect. Thus improving the chances of recovery for a patient. In some diseases caused by occlusion of blood vessels, the current perception is that there is no urgency in giving treatment since there is no effective way to prevent the tissue damage. A good example of such a disease is stroke. Very often, the fact that a patient had a stroke is not considered reason for immediate treatment the patient. Although with the advent of rtPA-treatment this is starting to change.
It is generally thought that damage inflicted by the stroke is already irreversible by the time a patient is hospitalized and available for treatment. With the present invention it will be apparent that there is a good reason for rapid treatment of patients. With the means and methods of the invention it is possible to at least in part reduce the extent of tissue damage. Also described herein is a method for at least in part preventing cell death in post-ischemic tissue comprising administering to an individual, having said tissue, β-interferon or a functional part, derivative and/or analogue thereof.

β-Interferon is currently used in the chronic treatment of MS patients and has been reported to cause systemic side-effects in this patient group. The systemic reaction is usually described as flu-like. Fever, chills, muscle aches, sometimes headaches, often commence a few hours after the drug is injected and last for some hours. In non-chronic cases administration of β-interferon can be discontinued after a relatively short period, often no longer than 1 week after the onset of the disease.

In the present invention the functionality of β-interferon or a functional part, derivative and/or analogue is used to at least in part prevent impairment of blood flow in post-ischemic tissue. A functional part of β-interferon is a part of β-interferon comprising the same post-ischemic damage reduction activity in kind as β-interferon itself. The amount of activity of such a part may differ from the activity of the complete protein. A person skilled in the art is capable of generating a suitable derivative of β-interferon. Derivatives can be for instance be obtained by conservative amino acid substitution, indeed currently prescribed human β-IFNs differ slightly in amino acid sequence from natural human β-IFN. A suitable part of β-interferon is for instance a part with a reduced amount of glycosylation. Glycosylation can be prevented by removing or altering a glycosylation site of the molecule. If the generation of such a (partially) deglycosylated β-interferon requires alteration of the amino acid composition than such a deglycosylated β-interferon is derivative of a functional part of β-interferon. A functional part, derivative and/or analogue of β-interferon comprises the same activity in kind not necessarily in amount.

In general, β-interferon may modulate the profile of cytokine production toward that of the anti-inflammatory phenotype (important are for example: increased IL-10, decreased IL-2, IFN-gamma and decreased TNF-alpha), and this appears to occur in the systemic circulation and within the CNS.

For suitable doses ranges and formulations of β-interferon or a functional part, derivative and/or analogue thereof one may turn to literature regarding the use of said compound in humans in other disease area's such as multiple sclerosis. Additionally, dose finding studies can give suitable therapeutic doses for the present invention. By way of example a dose of 8M IU Betaseron, s.c., every other day; the currently accepted dosage regime for relapsing remitting MS. A 1.6 M IU dosage every other day was well tolerated by the patient. [Biomed Pharmacother. 1999 Sep;53(8):344-50. Review]. [Biomed Pharmacother. 1999 Sep;53(8):344-50. Review].

The administration of β-interferon or a functional part, derivative and/or analogue thereof, can be combined with on or more other compounds. Preferably said compound blocks the upregulation of adhesion molecules on endothelium or on the cells they would bind. However antibody against these receptors, shielding it and preventing binding or a chemokine-antagonist that inhibits the activity of chemokines regulating adhesion molecule expression is also suitable. Also, some forms of IFN-alpha can have similar effects.

In a preferred embodiment β-interferon or a functional part, derivative and/or analogue thereof is combined with treatment given to remove an obstruction from a vessel. Preferably, said treatment comprises administration of rtPA or similar compound.

In another aspect the invention provides a use of β-interferon or a functional part, derivative and/or analogue thereof for the preparation of a medicament for the treatment of hypoxia-induced flow resistance. Also described herein is a use of β-interferon or a functional part, derivative and/or analogue thereof for the preparation of a medicament, said medicament capable of at least in part preventing cell death in post-ischemic tissue. Preferably, said impairment is in micro-vessels. More preferably, wherein said impairment is in the brain.

### Examples

### Materials and Methods

### Animal model

Male Fischer rats (F344/Ico, Iffa-Credo Broekman, Someren, The Netherlands) aged 8-12 weeks were used in all experiments. Animals had free access to standard laboratory chow and water. Anesthesia was induced by i.p. injection of a mixture of 0.5 ml/kg fentanyl citrate (0.315 mg/ml) and fluanisone (10.0 mg/ml), and 0.5 ml/kg midazolam (5.0 mg/ml), followed by s.c. injection of 0.1 ml/kg (0.5 mg/ml) atropine sulfate and i.p. injection of 0.5 ml/kg gentamicinsulfate (10 mg/ml). The animals were endotracheally intubated and mechanically ventilated with 02/N20 (30/70 v/v). Body temperature was maintained at 37.0 ± 0.5 °C by means of a feedback-controlled heating pad. During surgery and NMR-measurements anesthesia was continued by adding 1-1.5% halothane to the O₂/N₂O mixture. After surgery animals received 1 ml/kg buprenorfine s.c. (0.3 mg/ml).
Transient focal ischemia was induced by unilateral tandem occlusion of the right common carotid artery (CCA) and middle cerebral artery (MCA) via a modification of the procedure as described by Brint et al. (1988). Briefly, a 2 mm ∅ hole was drilled just rostral to the oval foramen, exposing the right middle cerebral artery (MCA). After opening the dura and arachnoidea the MCA was transiently occluded using a vascular microclip (Codman). Reperfusion was reinstated after 60 minutes under visual inspection by removing the clip.
Animals were treated with s.c. injections of 500.000 IU rrIFN-β in 1 ml phosphate buffered saline (PBS) once daily until 7 days after reperfusion (n=9). Control animals (n=5) received s.c injections with saline, starting 2 days prior to surgery up to 7 days after reperfusion. Treated animals were divided into three groups (n=3x3): treatment for group 1 started 2 days prior to surgery, treatment for group 2 started upon reperfusion and treatment for group 3 started 3 hours after reperfusion.

### NMR experiments

NMR-experiments were performed on a 4.7T Varian horizontal bore spectrometer equipped with a gradient insert able to achieve gradients up to 220 mT/m in 300 µs. Rf-excitation and signal detection were accomplished by means of a Helmholtz volume coil (9 cm ∅) and an inductively coupled surface coil (2 cm ∅), respectively. Animals were positioned in an animal cradle and immobilized with ear bars. During the experiments exhaled CO₂ and rectal temperature were continuously monitored.

A single-scan diffusion-trace MRI-sequence (4 b's:100-1780 s/mm², repetition time (TR)=2s, echo time (TE)=100 ms, number of transients (NT)=2) was used to generate quantified images of tissue water trace apparent diffusion coefficient (ADC). T₂-weighted images were acquired using a multi-echo sequence (8 TEs: 17.5 ms + 7 x 17.5 ms, TR=2s, NT=2).
Diffusion-weighted and T₂-weighted-data sets (collecting 8 1.7 mm thick slices, 3.2x3.2 cm² FOV, 128x64 matrix, zero-filled to 256x256) were acquired at 1, 7 and 21 days after reperfusion.

### Data analysis

ADC and T₂ maps were generated by mono-exponential fitting using IDL (Research Systems, Boulder, USA). Parametric images were analysed in anatomic regions of interest using in-house software. Calculations of volumes of affected tissue were based on ipsilateral ADC or T₂ differing more than 20% (corresponding to >2x SD) from the mean value in the contralateral hemisphere.
Statistical analysis was carried out using a linear mixed effects model (in S-PLUS 2000 Professional Edition Release 3, Mathsoft Inc., USA), with Rat as random effect and Day (the days the animals were scanned: 1, 7 and 21) and Treatment (control, beta-interferon-2 days prior, beta-interferon-upon reperfusion and beta-interferon-3 hours after reperfusion) as fixed effects. Different variances per Treatment were included in the model. No interaction between Day and Treatment was found; no main Day effect either. Treatment effect was extremely significant, due to difference between control and other treatments. No differences between other treatments were found.
Since there was no Day effect it was removed from further analyses. These were done using SPSS using two-tailed Student's t-tests or one-way ANOVA where appropriate.

### Results

### General Condition

After surgery, body weight of all animals showed an initial decrease in the first postoperative days, whereafter it increased.
In two treated animals a necrotic patch, about 7.5 mm in diameter, developed in the skin around the site of ifn-injection. The lesion was not painful to the touch and did not hinder the animals in feeding or other behavior. For the remaining days injection was continued at a different site, where, in these two animals, necrosis did not occur.

### Magnetic Resonance Imaging

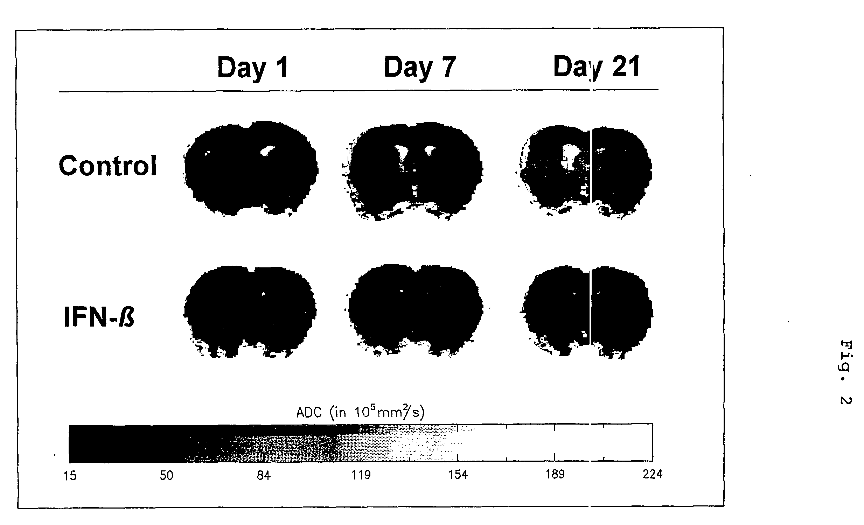

ADC images acquired at 1 day after reperfusion showed cytotoxic cell swelling, as evidenced by decreased ADC-values. In control animals both the cortex and a large part of the caudateputamen of the ipsilateral hemisphere were affected, while in interferon-treated animals a smaller part of the caudateputamen and no part or a only small rim of cortext showed cytotoxic cell swelling (see fig. 1). At this timepoint the volume of the lesion on ADC maps (ALV) was 76% smaller (p<0.0005) in treated animals compared to controls (see figs. 3 and 4). Also, in interferon-treated animals the severity of tissue damage, as determined from the severity of ADC-reduction, was 15% less (p<0.002) than in control animals (see fig. 5). There was no significant difference between the three treatment groups in terms of the T₂ data.
T₂ maps acquired at day 1 showed the development of vasogenic edema in the affected tissue, the volume of which was 64% smaller (p<0.021) in treated animals compared to controls (see figs. 2, 7 and 8). This difference is also demonstrated by the midline-shift caused by swelling of the brain tissue, which is absent or much less pronounced in treated animals compared to controls (see fig. 2). There was no significant difference between the three treatment groups.

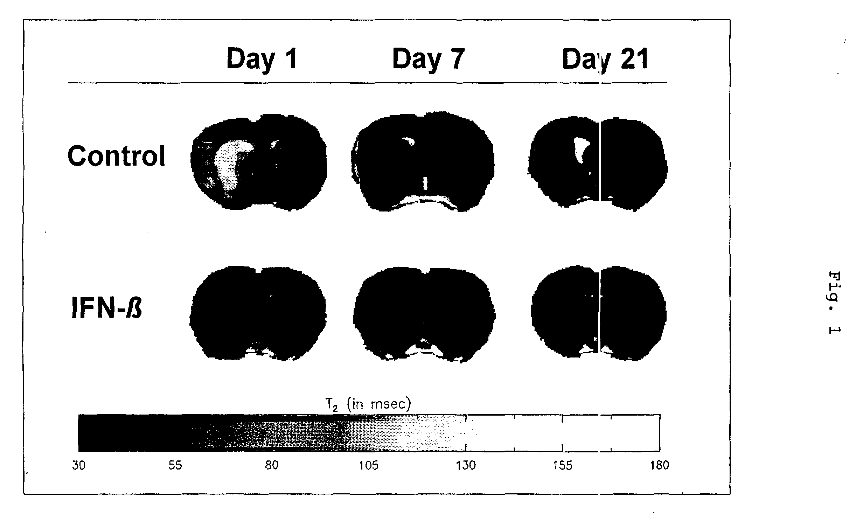

ADC data acquired during the endpoint measurement on day 21 showed ADC values had increased to supranormal values, indicative of vasogenic edema and tissue loss (see figs 1, 3 and 4). At this timepoint the infarct as present on ADC maps was reduced by 82% (p<0.0005) in treated animals compared to controls.
T₂ map data acquired on day 21 confirmed the presence of vasogenic edema and tissue loss (see figs 2, 7 and 8). Infarct size as determined from T₂ maps was 89% smaller (p<0.0005) in interferon treated animals compared to controls.
To summarize, treatment with interferon-â affords important neuroprotection to ischemic brain. Already at 1 day after reperfusion, the amount of tissue at risk to undergo permanent infarction identified by the ADC data, is significantly smaller in interferon-treated animals than in control animals. Permanent damage as assessed by T₂ MRI shows the protection afforded at day 1 extends until at least 3 weeks after the insult, at which timepoint the volume of infarcted tissue is 89% smaller in treated animals compared to controls.

There was no significant difference between starting treatment 2 days prior to the insult, starting upon reperfusion or starting 3 hours after reperfusion, the latest timepoint tested, indicating a treatment window of 4 hours after onset of stroke and possibly longer.

### Legends to the figures

**Fig 1. ADC maps of a slice through the center of the lesion acquired on day 1, 7 and 21.**
   Top row: Animal treated with s.c. injections of saline.
   Bottow row: Animal treated with s.c. injections of 500.000 IU rrIFN-*β*, starting at 3h after reperfusion.
**Fig 2. T₂ maps of a slice through the center of the lesion acquired on day 1, 7 and 21.**
   Top row: Animal treated with s.c. injections of saline.
   Bottow row: Animal treated with s.c. injections of 500.000 IU rrIFN-*β*, starting at 3h after reperfusion.

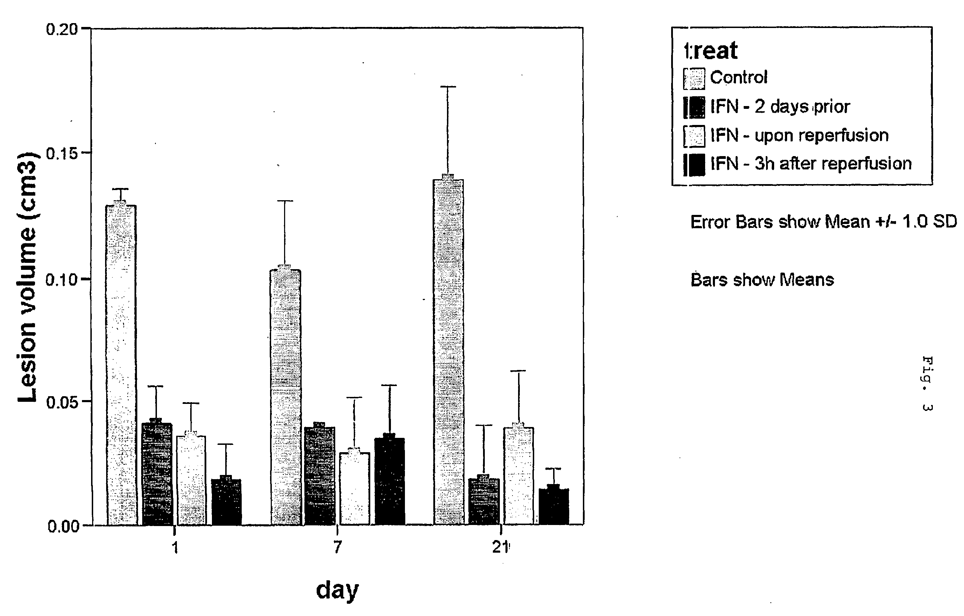

**Fig 3. Lesion volume of the control group and all treatment groups, on days 1, 7 and 21 as calculated from ADC maps.**

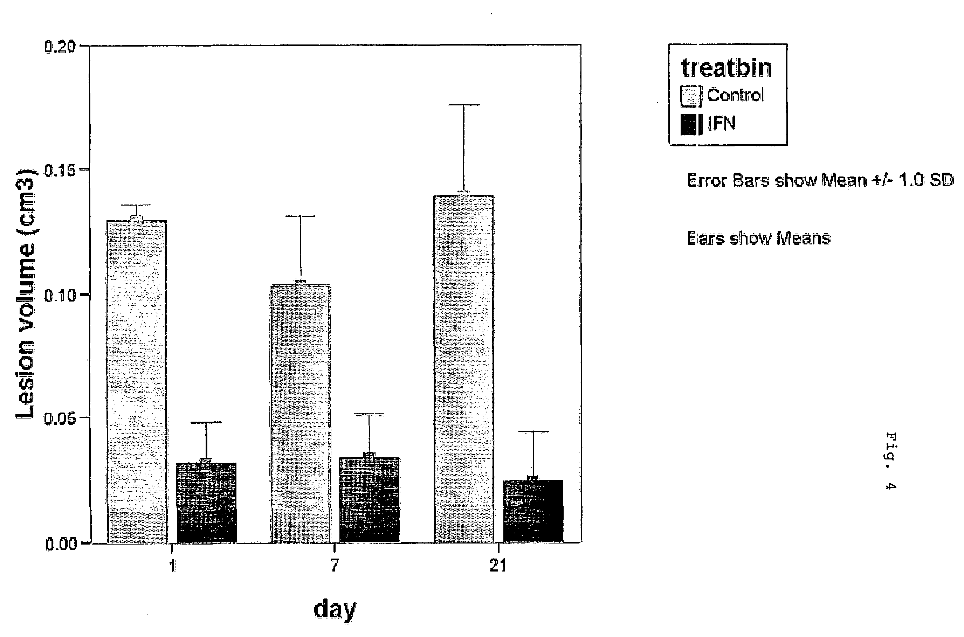

**Fig 4. Lesion volume of the control group versus all treatment groups together, on days 1, 7 and 21 as calculated from ADC maps.**

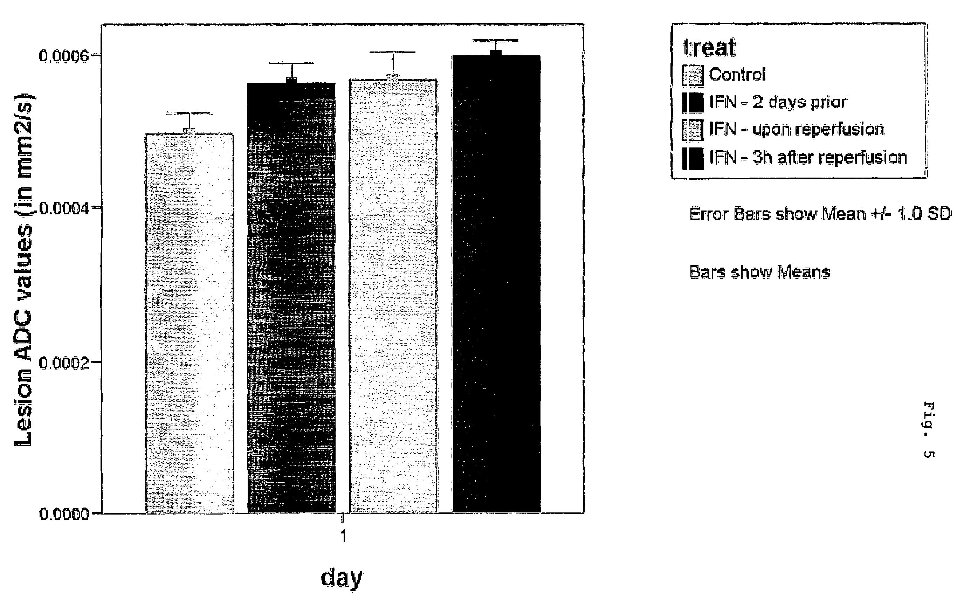

**Fig 5. Mean lesion ADC value of the control group and all treatment groups, on day 1 as calculated from ADC maps.** All values were decreased compared to normal tissue (0.00073 mm²/s); control animals showed a stronger decrease in lesion ADC value.

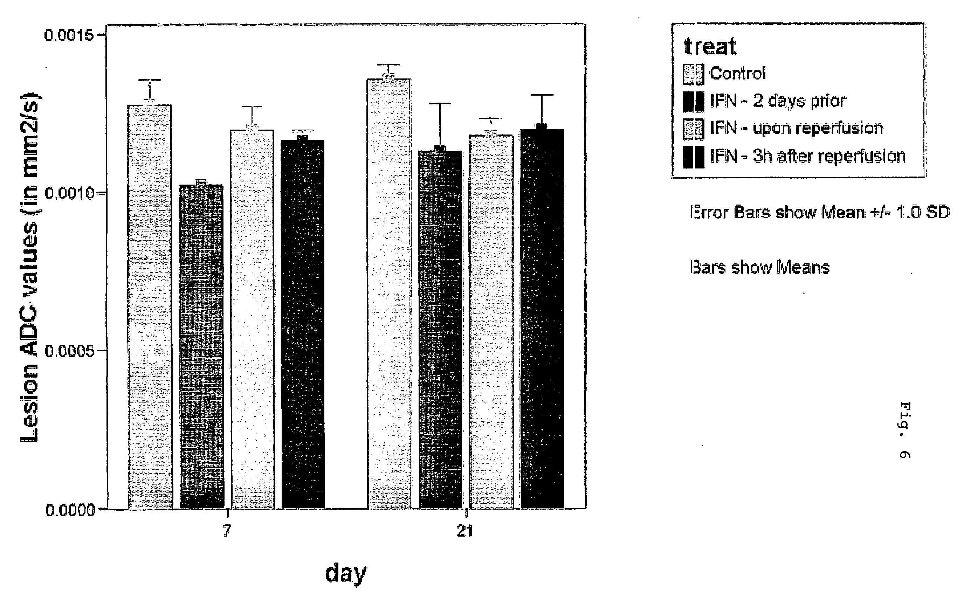

Fig 6. Mean lesion ADC value of the control group and all treatment **groups, on days 7 and 21 as calculated from ADC maps.** All values were increased compared to normal tissue (0.00073 mm²/s); control animals showed a stronger increase in lesion ADC value.

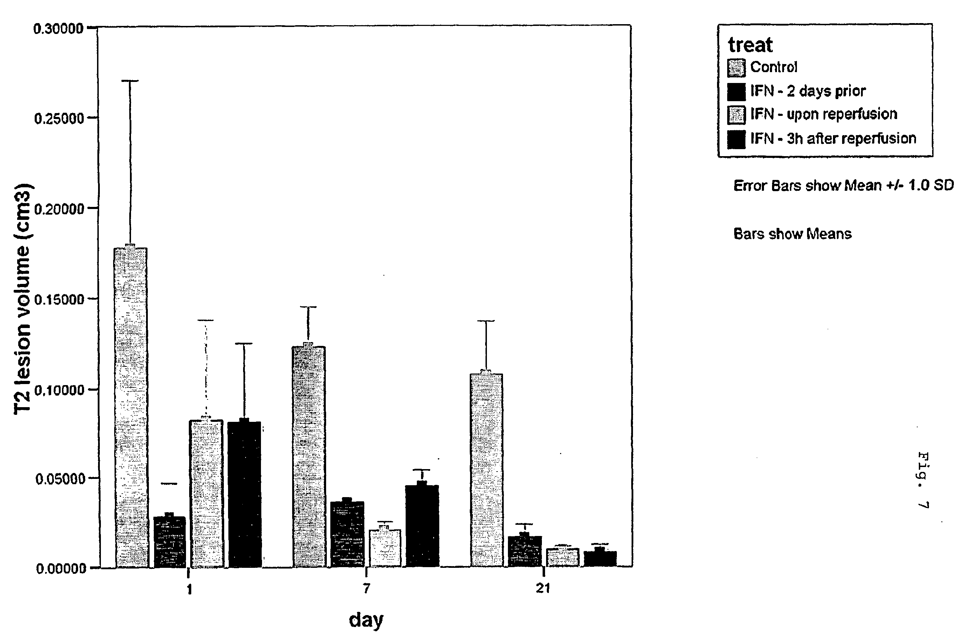

**Fig 7. Lesion volume of the control group and all treatment groups, on days 1, 7 and 21 as calculated from T₂ maps.**

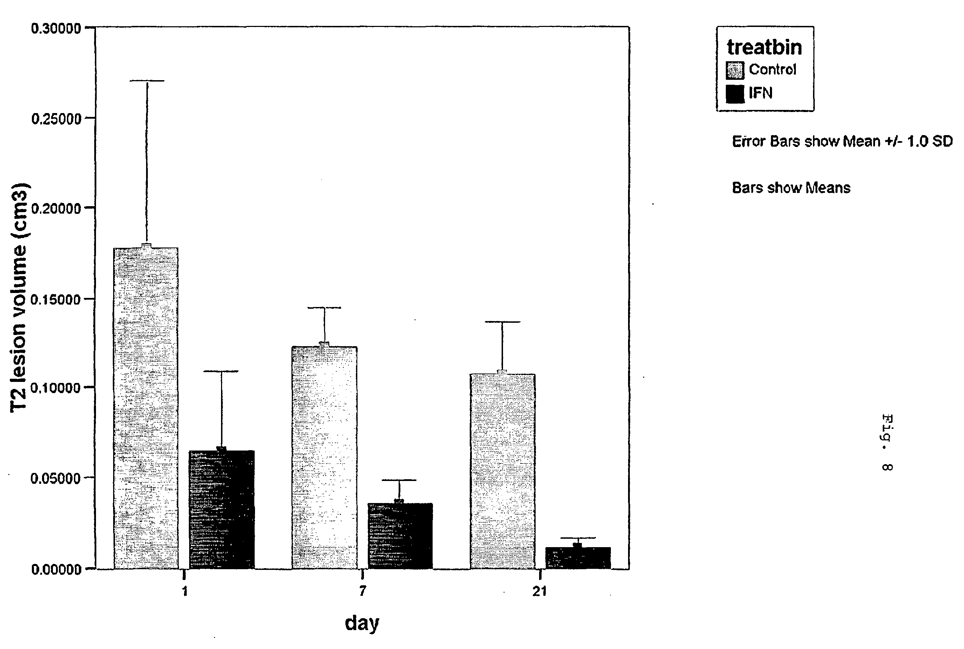

**Fig 8. Lesion volume of the control group versus all treatment groups together, on days 1, 7 and 21 as calculated from T₂ maps.**

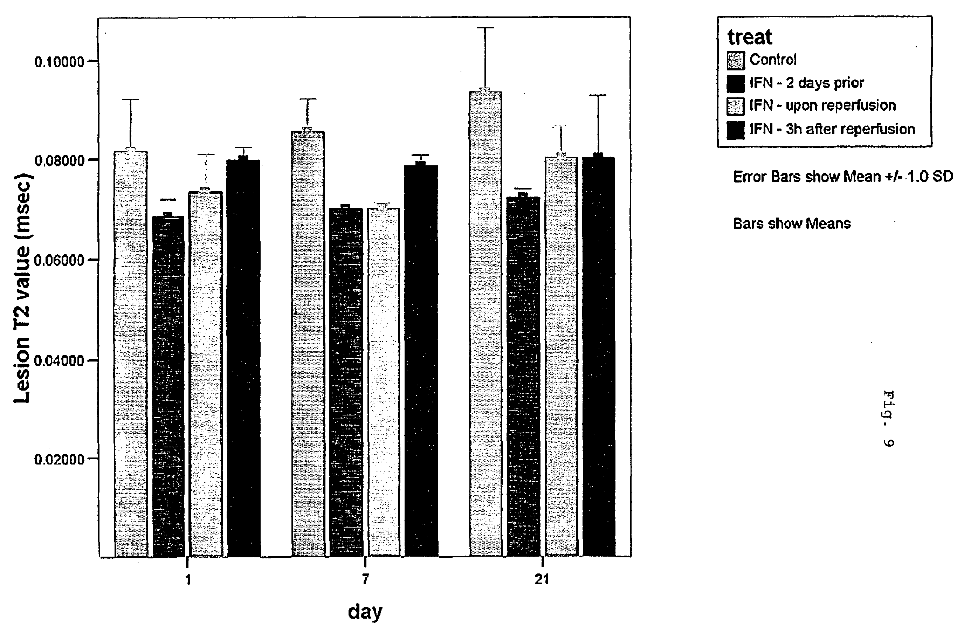

**Fig 9. Mean lesion T₂ value of the control group and all treatment groups, on days 1, 7 and 21 as calculated from T₂ maps.** All values were increased compared to normal tissue (0.055 s); control animals showed a stronger increase in lesion T₂ value.

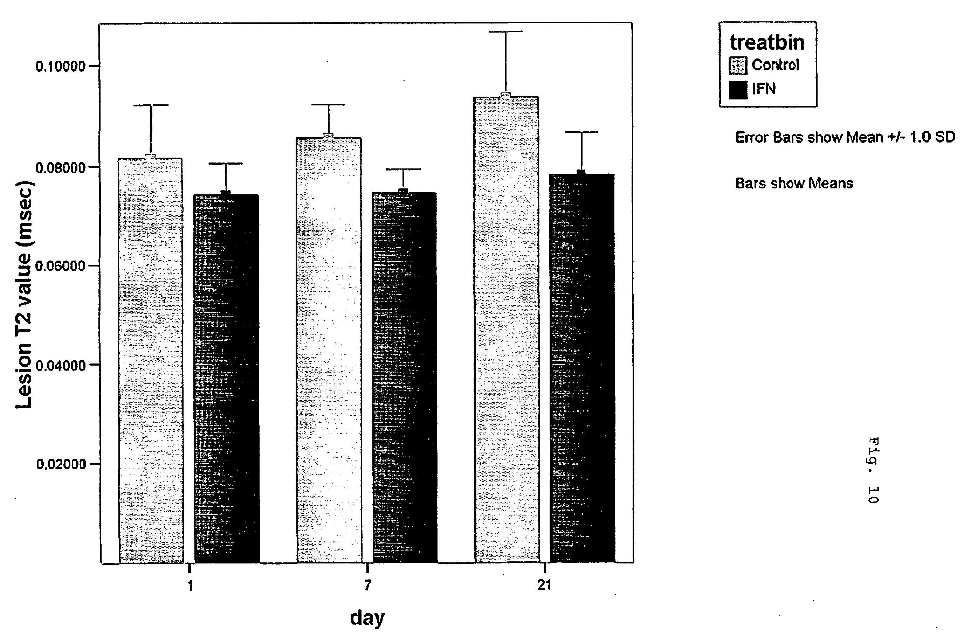

**Fig 10. Mean lesion T₂ value of the control group versus all treatment groups together, on days 1, 7 and 21 as calculated from T₂ maps.** All values were increased compared to normal tissue (0.055 s); control animals showed a stronger increase in lesion T₂ value.

### References

Brint S, Jacewicz M, Kiessling M, Tanabe J, Pulsinelli W (1988) Focal brain ischemia in the rat: methods for reproducible neocortical infarction using tandem occlusion of the distal middle cerebral and ipsilateral common carotid arteries. J Cereb Blood Flow Metab 8:474-485.
Biomed Pharmacother. 1999 Sep;53(8):344-50. Review
Biomed Pharmacother. 1999 Sep;53(8):344-50. Review

## Claims

1. Use of β-interferon or a functional part, derivative and/or analogue thereof, for the preparation of a medicament for the treatment of a hypoxia/ischaemia (H/I) related blood flow resistance in an individual.

2. Use according to claim 1, wherein said H/I related blood flow resistance is the result of a shortage in blood supply.

3. Use according to claim 1 or claim 2, wherein said H/I related blood flow resistance is restricted to a part of the body of said individual.

4. Use according to claim 3, wherein said part comprises the brain, the heart, a transplanted organ and/or limb.

5. Use according to claim 4, wherein said hypoxia related blood flow resistance is restricted to a part of the brain and/or the heart.

6. Use according to any one of claims 1-5, wherein said H/I related blood flow resistance is induced by an obstruction in an artery.

7. Use of β-interferon or a functional part, derivative and/or analogue thereof, for the preparation of a medicament for the treatment of a H/I related blood flow resistance in an individual, said individual comprising at least one blood vessel obstruction causing ischaemia in tissue that is situated downstream from said obstruction.

8. Use according to claim 1, wherein said blood flow resistance is correlated with ischaemia.

## Patentansprüche

1. Verwendung von β-Interferon oder eines funktionellen Teils, Derivats und/oder Analogons davon zur Herstellung eines Medikaments zur Behandlung eines mit Hypoxie/Ischämie (H/I) zusammenhängenden Blutstromwiderstands bei einem Individuum.

2. Verwendung gemäß Anspruch 1, wobei der mit H/I zusammenhängende Blutstromwiderstand das Ergebnis einer Knappheit in der Blutzufuhr ist.

3. Verwendung gemäß Anspruch 1 oder 2, wobei der mit H/I zusammenhängende Blutstromwiderstand auf einen Teil des Körpers des Individuums eingeschränkt ist.

4. Verwendung gemäß Anspruch 3, wobei der Teil das Gehirn, das Herz, ein transplantiertes Organ und/oder eine transplantierte Gliedmaße umfasst.

5. Verwendung gemäß Anspruch 4, wobei der mit Hypoxie zusammenhängende Blutstromwiderstand auf einen Teil des Gehirns und/oder des Herzens eingeschränkt ist.

6. Verwendung gemäß einem der Ansprüche 1 bis 5, wobei der mit H/I zusammenhängende Blutstromwiderstand durch eine Verstopfung in einer Arterie induziert ist.

7. Verwendung von β-Interferon oder eines funktionellen Teils, Derivats und/oder Analogons davon zur Herstellung eines Medikaments zur Behandlung eines mit H/I zusammenhängenden Blutstromwiderstands bei einem Individuum, wobei das Individuum wenigstens eine Blutgefäßverstopfung umfasst, die eine Ischämie in Gewebe verursacht, das sich stromabwärts der Verstopfung befindet.

8. Verwendung gemäß Anspruch 1, wobei der Blutstromwiderstand mit Ischämie korreliert ist.

## Revendications

1. Utilisation de β-interféron ou d'une partie fonctionnelle, d'un dérivé et/ou analogue de celui-ci, pour la préparation d'un médicament pour le traitement d'une résistance à la circulation sanguine apparentée à une hypoxie/ischémie (H/I) chez un individu.

2. Utilisation selon la revendication 1, dans laquelle ladite résistance à la circulation sanguine apparentée à H/I est le résultat d'un manque d'apport sanguin.

3. Utilisation selon la revendication 1 ou 2, dans laquelle ladite résistance à la circulation sanguine apparentée à H/I se limite à une partie du corps dudit individu.

4. Utilisation selon la revendication 3, dans laquelle ladite partie comprend le cerveau, le coeur, un organe et/ou membre greffé.

5. Utilisation selon la revendication 4, dans laquelle ladite résistance à la circulation sanguine apparentée à une hypoxie se limite à une partie du cerveau et/ou du coeur.

6. Utilisation selon l'une quelconque des revendications 1 à 5, dans laquelle ladite résistance à la circulation sanguine apparentée à H/I est induite par l'occlusion d'une artère.

7. Utilisation de β-interféron ou d'une partie fonctionnelle, d'un dérivé et/ou analogue de celui-ci, pour la préparation d'un médicament pour le traitement d'une résistance à la circulation sanguine apparentée à H/I chez un individu, ledit individu comprenant au moins une occlusion de vaisseau sanguin responsable d'une ischémie dans le tissu qui se trouve en aval de ladite occlusion.

8. Utilisation selon la revendication 1, dans laquelle ladite résistance à la circulation sanguine est liée à une ischémie.
